# EUROPEAN PATENT SPECIFICATION

(11) **EP 0 596 172 B1**
(45) Date of publication and mention of the grant of the patent: **07.07.1999**
(21) Application number: 92311736.0
(22) Date of filing: 23.12.1992
(51) Int. Cl.: A61M 25/01, A61M 25/06, A61M 39/06, A61M 25/00

(54) **Radiopaque non-kinking thin-walled introducer sheath**
Strahlungsundurchlässige nichtknickbare dünnwandige Einführvorrichtung
Introducteur à parois minces, radio-opaque, ne vrillant pas

(30) Priority: 03.11.1992 US 965961
(43) Date of publication of application: 11.05.1994
(73) Proprietor: Fischell, Robert E., Dayton, Maryland 21036 (US); Fischell, Tim A., Nashville, Tennessee 37205 (US)
(72) Inventor: Fischell, Robert E., Dayton, Maryland 21036 (US); Fischell, Tim A., Nashville, Tennessee 37205 (US)
(74) Representative: Harris, Ian Richard

(56) References cited:
- EP-A- 0 145 489
- EP-A- 0 530 970
- WO-A-90/02579
- US-A- 4 411 655
- US-A- 4 634 432
- US-A- 5 092 848

## Description

This invention relates to introducer sheaths that are inserted through the skin into an artery or other vessel in a living body for the purpose of subsequent percutaneous insertion and guidance of a transluminal catheter.

It is common practice in the fields of angioplasty and atherectomy to insert catheters into the artery through a plastic sheath. These sheaths are typically made from PVC or an equivalent plastic and typically have a wall thickness of 0.254mm (10 mils) it being highly advantageous to have those sheaths as thin-walled as possible. The inner diameter of such sheaths is determined by the diameter of the catheter to be placed through it, so that in order to provide that 0.254mm thickness the external diameter of the sheath is typically 0.508mm (20 mils) greater than the inside diameter. However, it would be highly advantageous to reduce the outside diameter of the sheath so as to minimise arterial distention, thereby reducing the bleeding that occurs at the insertion side after the catheter and sheath are removed from the artery, but against that significant problems do arise from the tendency of extremely thin-walled sheaths to kink or bend at the point of entry into, for example, the femoral artery or where they pass through a highly curved section of an iliac artery. Also because they are made from plastics materials, the current thin-walled sheaths are not significantly radiopaque.

In accordance with the invention, there is provided an introducer sheath for percutaneous insertion into a vessel of a human or animal body and when so inserted serving as a guide for the subsequent insertion of a catheter into said vessel, the sheath comprising an adapter located at one end of the sheath to allow the insertion of guide wires and/or catheters through the sheath and into the said vessel, and an introducer sleeve comprising a wire metal coil of flat wire strip which forms at least part of the interior wall of the sleeve, the strip being coated or covered with a plastic covering that is fitted onto and is in contact with the exterior surface of said metal coil, characterised in that: said adapter comprises a hemostasis valve; and said wire metal coil is formed with spaces betwen adjacent turns thereof and said plastics covering has portions thereof extending into said spaces a distance sufficient to resist relative longitudinal displacement of said adjacent turns when said sheath is bent but without covering the interior surface of the metal coil.

Embodiments of the present invention seek to address the shortcomings of the prior art devices by providing a radiopaque sheath that is non-kinking and with a thinner wall as compared with sheaths that are currently available. This may be achieved by inserting into the sheath a helical metal coil preferably fabricated from flat wire or by utilising as the sheath such a helical metal coil covered or coated with a thin plastic covering. This may be provided by means of a plastic material coated onto and between the turns of the metal coil or by using a length of heat shrinkable tubing, or by moulding or extruding plastic over the thin helical metal coil. At the proximal end of the sheath an adapter (hemostasis valve) may be provided through which the catheter may subsequently be placed. This adapter would typically be moulded from a plastic so as to both join onto the metal coil as well as mould onto the plastic covering of the metal coil. At its distal end, the sheath would advantageously combine a metal portion for radiopacity and at its extreme end a soft plastic tapered end piece.

EP-A-0,530,970 discloses an introducer sheath comprising an inner tube, a wire coil wound upon the tube and a sleeve provided over the coil. EP-A-0,350,970 is only citable under Article 54(3) EPC.

US-A-4,411,655 discloses an introducer sheath comprising a helical coil which is covered by a plastics sleeve. The coil and sleeve are dilatable during use.

US-A-4,634,432 discloses an introducer sheath comprising a connector body and a flexible sleeve, the sleeve having a metal coil embedded therein. The coil being entirely surrounded by the sleeve.

The invention will be further described by reference to the accompanying drawings, in which:
Fig. 1 is a cross-sectional view of a non-kinking, thin-walled sheath according to an embodiment of the invention and comprising single helical metal coil having a plastics covering and with plastic extensions between adjacent turns.
Fig. 2A shows a wall section of a sheath in accordance with the invention, with a plastic extension incompletely filling the space between adjacent turns.
Fig. 2B shows a wall section of a sheath in accordance with the invention where the spaces between the adjacent turns of the coil are completed filled.
Fig. 2C shows a wall section of a sheath in accordance with the invention where the spaces between the adjacent turns of the coil are almost filled.
Fig. 2D shows a wall section of a sheath in accordance with the invention wherein the spaces between adjacent turns of the coil are overfilled.
Fig. 3A shows a wall section of a sheath in accordance with the invention wherein the flat wire is considerably thinner than the plastic covering and there is a greater spacing between adjacent turns.
Fig. 3B shows a wall section of a sheath in accordance with the invention wherein only the inner corners of the flat wire metal coil are rounded.
Fig. 3C shows a wall section of a sheath in accordance with the invention wherein only the inner corners of the flat wire metal coil are chamfered.
Fig. 4 is a longitudinal cross section of the distal end of the sheath according to the invention illustrating a metal tip.

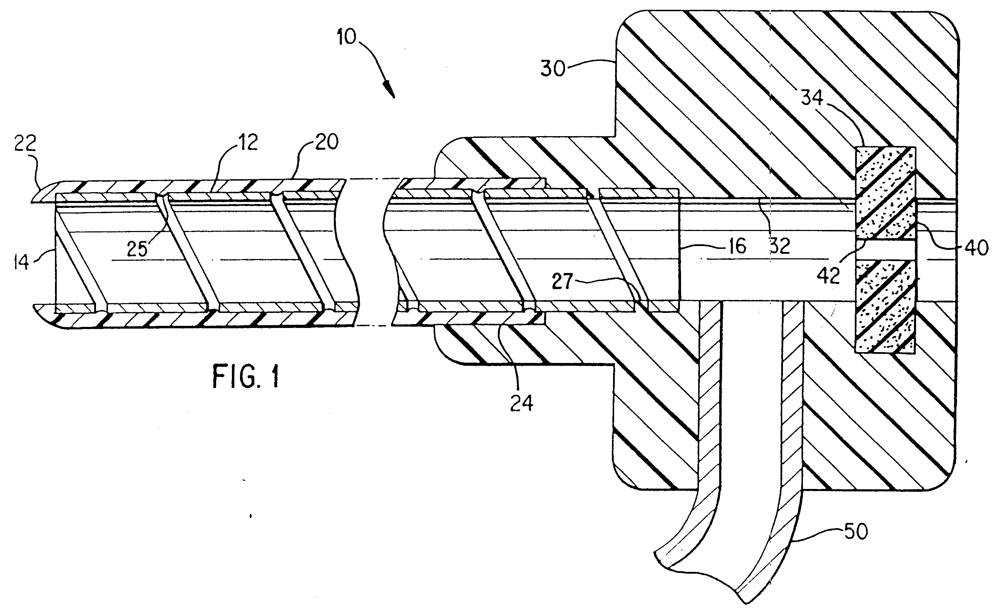

Referring first to Fig. 1, a non-kinking, thin-walled sheath 10 is shown with an inner metal coil 12 that lies within a plastic covering 20 for most of the length of the sheath with a plastic adapter 30 moulded onto the proximal end of the sheath 10. The metal coil 12 would typically be fabricated from flat stainless steel wire or an equivalent springy metal. Metals such as 300 or 400 series stainless steel, nickel alloys such as Monel® metal, or Inconel® or beryllium copper, tantalum or gold alloys could be used for the flat wire metal helix material. The thickness of the wire would typically be in the range 0.0254 to 0.127mm (1 to 5 mils) and the width of the wire would typically be between 3 to 80 times the wire thickness. This ratio of wire width to wire thickness is a very important consideration in the design of the sheath in order to prevent the sheath from collapsing while still providing a very thin wall. By actually building a model of this sheath it has been determined that for the most desirable wire thicknesses which provide a very thin wall while reducing the likelihood of the sheath collapsing in normal handling, only certain ranges of wire width to wire thickness are reasonable and these ratios are presented in Table 1. This table clearly shows greater width-to-thickness ratios are required as the wire thickness is decreased.

**Table 1**

| **Acceptable Wire Width-To-Thickness Ratios As a Function of Wire Thickness** | |
|---|---|
| **THICKNESS RANGE mm(mils)** | **RANGE OF WIDTH-TO-THICKNESS RATIO** |
| 0.0635 to 0.0889 (2.50 to 3.50) | 3:1 to 20:1 |
| 0.0381 to 0.0632 (1.50 to 2.49) | 5:1 to 50:1 |
| 0.0190 to 0.0378 (0.75 to 1.49) | 12:1 to 80:1 |

The flat wire helix would typically be wound on a mandrel in a similar manner to the way that spring wire guides are made at the present time. Another method to form the flat wire helix would be by using machines than form tension or compression coil springs. The inner diameter of the helical coil would typically lie in the range 1.016 to 5.08mm (40 to 200 mils) depending on the size of the catheter that has to be inserted through it. The distal end 14 and the proximal end 16 of the coil 12 would be typically cut off square as shown in Fig. 1.

Covering the helical coil 12 would be a plastic covering 20 having a thickness in the 0.0254 to 0.203mm range (1 to 8 mils) and which would typically be made from polyethylene, polyurethane, PVC, Surlyn® or a similar plastic material. One method for forming the covering 20 so that it fits tightly around the helical coil 12 would be by sliding the coil 12 through a tube of the plastic and then heat shrinking the plastic onto the helical coil 12. Another method would be to dip coat the coil 12 into a liquid plastic material that hardens onto the helical coil 12 after dipping. Another method would be to overextrude plastic over the coil 12. A polytetrafluoroethylene (Teflon: Registered Trade Mark) mandrel could be inserted inside the metal coil 12 before dip coating or overextruding. Whatever method is used to form the plastic covering 20, the plastic material could have a partially filled extension 25 or a fully filled extension 27 each of which projects into the space between adjacent turns thereby preventing unwanted longitudinal displacement of one turn relative to another when the sheath 10 is severely bent. The inside diameter of either extension 25 or 27 is smaller than the outside diameter of the metal coil 12 and larger than or equal to the inside diameter of the metal coil 12.

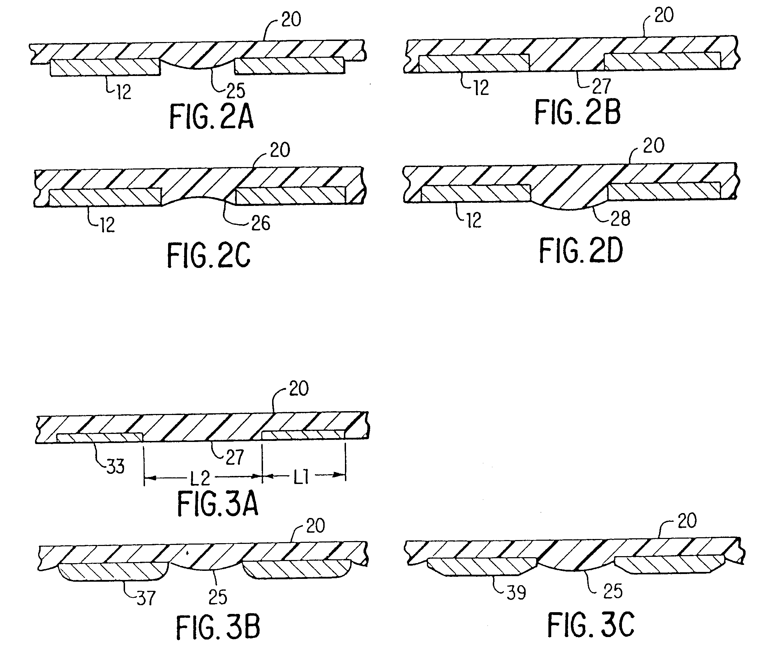

Figs. 2A, 2B, 2C and 2D show four sheath wall sections having four different types of plastic extensions that keep adjacent turns of coils separated from each other.

Fig. 2A is an enlarged view of the wall section of the sheath shown in Fig. 1 which has a partially filled extension 25 protruding from the plastic covering 20 which extension only slightly fills the space between adjacent turns. However, the sharp corners of the metal coil 12 prevents unwanted longitudinal displacement of the turns of the coil when the sheath is bent. This form of plastic extension is typical of that which would be obtained with heat shrinkable tubing followed by centreless grinding of the outer surface of the covering 20.

Fig. 2B shows a completely filled extension 27 of the plastic covering 20 which design is also shown in Fig. 1. This shape would typically be obtained when the plastic covering 20 is overextruded with a tight fitting cylindrical mandrel (typically made from a polytetrafluoroethylene cylinder) placed inside the metal coil 12. The tight fitting mandrel (not shown) prevents plastic from adhering to the inner surface of the metal which, if it should occur, would result in an undesired increased wall thickness of the sheath. This type of projection could also be obtained by placing a liquid plastic material between the turns of a metal coil that has been wound on a mandrel and then placing an outer plastic covering 20 over the metal coil 12. An importance of this design is that the sharp inner corners of the flat wire are covered.

Fig. 2C shows a mostly filled extension 26 which could be formed by placing a hollow polytetrafluoroethylene (Teflon) tube (not shown) inside the metal coil and then inflating the tube and then overextruding the plastic covering 20 onto and in between the coil 12. The tube would be deflated to allow it to be withdrawn. An importance of this design (like Fig. 2B) is that the sharp inner corners of the flat wire are covered.

Fig. 2D shows an over-filled extension 28 which could be formed by using heat shrinkable tubing for the plastic covering 20 and then heating the metal coil 12 until the metal coils "melt" into that plastic covering 20. It is also possible to overextrude the plastic covering 20 and with the appropriate type of plastic, pressure and temperature to form the plastic shape as shown in Fig. 2D. This design covers the inner corners of the flat wire and furthermore, an inner lubricity coating could be applied to the plastic to allow easier passage for an inserted catheter.

Figs. 3A, 3B and 3C illustrate three other embodiments of wall sections for a non-kinking sheath. Fig. 3A shows an embodiment in which the flat wire metal coil 33 has a wire thickness that is considerably smaller than the thickness of the plastic covering 20. This particular wall section is shown with a plastic extension 27 similar to that shown in Fig. 2B. The width of each turn of the coil 33 is L1, and the length of the separation between turns is L2. In Fig. 3A, L2 is greater than L1. Typically L2 would be equal to or less than L1. However, if greater flexibility is desired, L2 can be several times greater than L1. However, if L2 is greater than 1 to 2 cm, then the sheath might no longer be non-kinking. It should also be understood that a sheath might use a variable spacing L2 between adjacent turns. For example, L2 might be 0.5mm for most of the sheath's length but L2 might be gradually increased to 5mm at the sheath's distal end in order to increase the flexibility of the sheath's distal end.

Figs. 3B and 3C show an embodiment of the coils 37 and 39 in which there is a generally squared off outer corner at the end of each turn and a generally rounded inner corner at the end of each turn. Specifically, in Fig. 3B the inner corners of the coil 37 are rounded and in Fig. 3C the inner corners of the turn 39 are chamfered. It should also be understood that the outer surface of the coil could be finished so as to prevent adhesion of the plastic covering 20 to the coil; or conversely, the outer surface of the coil could be treated to cause the metal coil to bond to the plastic covering 20. Generally, adhesion or bonding of the coils outer surface to the plastic covering 20 will result in a less flexible sheath. Furthermore, increasing the ratio of L2/L1 (as seen in Fig. 3A) will increase sheath flexibility. It is well known in the wire forming art that any of the wire shapes showing in Fig. 3 could be obtained by slitting, drawing or extruding the flat wire through a die; or a combination of these methods could be used to form the desired cross section of the generally flat wire. The cross section of Fig. 3A could also be obtained by rolling down round wire.

The shapes shown in Figs. 3B and 3C are advantageous in that their sharp outside corners dig into the plastic covering 20 thus preventing unwanted longitudinal displacement of the turns of the coil when the sheath is severely bent. Furthermore, the rounding or chamfering of the inside edge prevents the outer surface of a tight fitting catheter from being damaged by exposed sharp inner corners such as those shown in Fig. 2A as a tight fitting catheter is pushed through the sheath. Also, a tight fitting catheter would slide through the inside of the sheath with less friction or catching (especially through bends in the sheath) if the inside corners of the metal coil are rounded or chamfered as shown in Figs. 3B and 3C.

It also envisioned that any of the flat wire metal coil designs described herein could be coated with a metal or plastic so as to enhance the sheath's radiopacity or to decrease frictional forces on any catheter that would be placed through the sheath. For example, gold or tantalum plating of the flat wire would enhance the sheath's radiopacity. Furthermore, the metal coil could have a lubricating coating applied to decrease frictional forces of objects passing through the sheath's interior lumen. Additionally, the bare metal could be given a thin plastic coating which would then have a lubricating coating applied. Further, as to coatings, a lubricating coating could be applied to the sheath's exterior plastic covering 20 to allow the sheath to enter human tissue and advance through human blood vessels while minimising frictional resistance. The outer surface of the plastic covering 20 could also be treated with an anti-bacterial coating which would be especially important for sheaths that remain in a vessel for more than a few hours. Still further, the exterior plastic covering 20 could be centreless ground to make a smoother outer surface of the sheath.

In Fig. 1 we see that the distal tip 22 of the plastic covering 20 might be heat moulded to an appropriate shape which can readily pass through the arterial wall with the aid of a dilator (not shown). The proximal end 24 of the covering 20 would have moulded onto it a plastic adapter 30 (typically including a hemostasis valve) which can have a side-port 50 as shown in Fig. 1. The adapter 30, which may be formed from the same plastic material as the covering 20 or from another material such as PVC, would also be moulded onto the proximal end of the helical coil 12. The adapter 30 would have an interior cylindrical hole 32 whose inside diameter is moulded to match the inside diameter of the helical coil 12. A cylindrical groove 34 would be moulded into the adapter 30 so as to accept a foam rubber packing gland or hemostasis valve 40. The packing gland 40 has a hole 42 through its centre to allow for the passage of a catheter. The purpose of the gland 40 is to seal around the outside diameter of the catheter when it is in place to prevent arterial blood from escaping between the inner cylinder 32 of the adapter 30 and the outside diameter of the catheter that is percutaneously placed into the arterial system. The packing gland 40 is only indicative of more sophisticated hemostasis valves that would be used with such a sheath. An example of such a valve is shown in U.S. Patent No. 5,041,095 by P.K. Littrell entitled "Hemostasis Valve".

As previously described, the strip of metal forming the helical coil 12 would have a thickness in the range 0.0254 to 0.127mm (1 to 5 mils). Similarly the plastic covering 20 would typically have a thickness in the same range to that the total thickness of the coil 12 and covering 20 would be in the range 0.0508 to 0.254mm (2 to 10 mils). At 0.254mm thickness, the sheath would have the advantage of being non-kinking and radiopaque. However, it would not have any advantage in reducing the outer diameter of the sheath 10 as compared to other sheaths that are currently available. However, as we approach wire and plastic covering thicknesses the order of 0.0508mm (2 mils), the outer diameter of the sheath 10 is significantly reduced. There is a distinct advantage in dramatically reducing the wall thickness of the sheath 10 while at the same time having improved resistance to kinking which is provided by the strength of the helical coil 12.

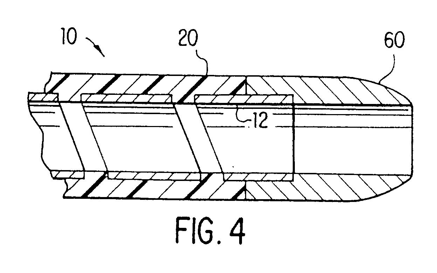

Fig. 4 illustrates an improved tip design for this type of thin-walled sheath 10. As typical for this sheath design, the metal coil 12 is encased in a plastic covering 20. A metal tip 60 is joined to the coil 12 and/or covering 20 by adhesive bonding, welding or brazing or an equivalent joining means. Although a stainless steel tip could be used, a dense metal such as gold or tantalum (or an alloy of these metals) would have the advantage of greater radiopacity.

Although Fig. 1 shows only a single coil 12, it is envisioned that in a second embodiment, the helical coil 12 might be made from two separate metal coils, one inside the other, that are wound in opposite directions so as to improve the strength of the sheath.

All the sheaths designs described herein have metal coils which are intrinsically radiopaque. Hence these sheath designs have the additional functional attribute of being radiopaque even without the addition of highly radiopaque distal tips.

Although the utilisation of sheaths in arteries is described herein in considerable detail, the sheath that is taught herein is also able to be used for access to a variety of lumens of humans or animals, such as veins, urethras, fallopian tubes, biliary ducts, bronchial tubes or any similar vessel in a living body.

Various other modifications, adaptions, and alternative designs are of course possible in light of the above teachings without departing from the scope of the invention.

## Claims

1. An introducer sheath (10) for percutaneous insertion into a vessel of a human or animal body and when so inserted serving as a aide for the subsequent insertion of a catheter into said vessel, the sheath (10) comprising an adapter (30) located at one end of the sheath to allow the insertion of guide wires and/or catheters through the sheath and into said vessel, and an introducer sleeve (12,20) comprising a wire metal coil (12) of flat wire strip which forms at least part of the interior wall of the sleeve, the strip being coated or covered with a plastic covering (20) that is fitted onto and is in contact with the exterior surface of said metal coil, characterised in that:
said adapter (30) comprises a hemostasis valve; and
said wire metal coil (12) is formed with spaces between adjacent turns thereof and said plastics covering (20) has portions thereof (25,26,27,28) extending into said spaces a distance sufficient to resist relative longitudinal displacement of said adjacent turns when said sheath is bent but without covering the interior surface of the metal coil.

2. An introducer sheath according to claim 1 or 2, wherein the metal coil (12) is made from stainless steel.

3. An introducer sheath according to claim 1 or claim 2, wherein the flat wire of the metal coil has a thickness in the range 0.0635 to 0.0889mm (2.5 to 3.5 mils) and a width to thickness ratio to the range 3:1 to 20:1, or a thickness in the range 0.0381 to 0.0632mm (1.50 to 2.49 mils) and a width to thickness ratio in the range 5:1 to 50:1, or a thickness in the range 0.019 to 0.0378mm (0.75 to 1.49 mils) and a width-to-thickness ratio in the range 12:1 to 80:1.

4. An introducer sheath according to any of claims 1 to 3, wherein the spaces between adjacent turns of the coil are each less than the width of a single turn of the coil.

5. An introducer sheath according to any of claims 1 to 3, wherein the spaces between adjacent turns of the coil are larger than the width of a single turn of the coil.

6. An introducer sheath according to claim 4 or 5, wherein the spaces between adjacent turns are all of the same width.

7. An introducer sheath according to claim 4 or 5 wherein the width of the spaces between adjacent turns varies along the length of the coil.

8. An introducer sheath according to claim 7, wherein the width of the spaces between adjacent turns at the sheath's distal end are greater than those at the proximal end adjacent said adapter (30).

9. An introducer sheath according to any preceding claim, wherein the flat wire strip has chamfered or rounded edges.

10. An introducer sheath according to claim 9, wherein the chamfered or rounded edges of the wire strip in each turn of the coil are substantially contiguous with those of the adjacent turns.

11. An introducer sheath according to any of claims 1 to 10, wherein the metal coil (12) is plated with a dense radiopaque metal.

12. An introducer sheath according to any of claims 1 to 11, wherein the internal surface of the metal coil is provided with a lubricating coating.

13. An introducer sheath according to any of the preceding claims wherein the outer covering (20) of the sheath comprises a preformed plastics sleeve, heat shrunk onto the metal coil (12).

14. An introducer sheath according to any of the preceding claims wherein the thickness of the plastic covering is in the range 0.0254 to 0.203mm (0.001 and 0.008 inches).

15. An introducer sheath according to any of the preceding claims wherein the plastic covering has a smooth finish on its exterior surface, the finish being provided by centreless grinding of the exterior surface.

16. An introducer sheath according to any of the preceding claims wherein the plastic covering has a lubricated external surface.

17. An introducer sheath according to any of the preceding claims wherein the sheath has a metal tip (60) at the end.

18. An introducer sheath according to claim 17, wherein the metal tip (60) consists of or comprises a high density radiopaque metal.

19. An introducer sheath according to claim 11 or claim 18, wherein the radiopaque metal is tantalum or gold.

## Patentansprüche

1. Einführhülse (10) für perkutanes Einführen in ein Gefäß eines menschlichen oder tierischen Körpers, welche nach diesem Einführen als Führung für die anschließende Einführung eines Katheters in das Gefäß dient, wobei die Hülse (10) einen Adapter (30) aufweist, der an einem Ende der Hülse angeordnet ist, um das Einführen von Führungsdrähten und/oder Kathetern durch die Hülse und in das Gefäß zu erlauben, und eine Einführmuffe (12, 20), welche eine Drahtmetallspule (12) aus flachem Drahtstreifen aufweist, der mindestens einen Teil der Innenwand der Muffe bildet, wobei der Streifen mit einer Kunststoffumhüllung (20) beschichtet oder umhüllt ist, die auf der äußeren Oberfläche der Metallspule eingerichtet und mit dieser in Kontakt ist, dadurch gekennzeichnet, daß:
der Adapter (30) ein hämostatisches Ventil aufweist; und
die Drahtmetallspule (12) mit Räumen zwischen ihren nebeneinanderliegenden Windungen gebildet ist und die Kunststoffumhüllung (20) Abschnitte (25, 26, 27, 28) hat, die sich in die Räume um einen ausreichenden Abstand erstrecken, um der relativen Längsverschiebung der nebeneinanderliegenden Windungen zu widerstehen, wenn die Hülse gebogen wird, aber ohne die innere Oberfläche der Metallspule zu belasten.

2. Einführhülse nach Anspruch 1 oder 2, wobei die Metallspule (12) aus nicht rostendem Stahl hergestellt ist.

3. Einführhülse nach Anspruch 1 oder 2, wobei der flache Draht der Metallspule eine Dicke in dem Bereich von 0,0635 bis 0,0889 mm (2,5 bis 3,5 mils) und ein Breiten-zu-Dicken-Verhältnis in dem Bereich von 3:1 bis 20:1 hat oder eine Dicke in dem Bereich von 0,0381 bis 0,0632 mm (1,50 bis 2,49 mils) und ein Breiten-zu-Dicken-Verhältnis in dem Bereich von 5:1 bis 50:1 oder eine Dicke in dem Bereich von 0,019 bis 0,0378 mm (0,75 bis 1,49 mils) und ein Breiten-zu-Dicken-Verhältnis in dem Bereich von 12:1 bis 80:1.

4. Einführhülse nach einem der Ansprüche 1 bis 3, wobei die Räume zwischen nebeneinanderliegenden Windungen der Spule jeweils kleiner sind als die Breite einer einzigen Windung der Spule.

5. Einführhülse nach einem der Ansprüche 1 bis 3, wobei die Räume zwischen nebeneinanderliegenden Windungen der Spule größer sind als die Breite einer einzelnen Windung der Spule.

6. Einführhülse nach Anspruch 4 oder 5, wobei die Räume zwischen nebeneinanderliegenden Windungen alle dieselbe Breite haben.

7. Einführhülse nach Anspruch 4 oder 5, wobei die Breite der Räume zwischen nebeneinanderliegenden Windungen entlang der Länge der Spule variiert.

8. Einführhülse nach Anspruch 7, wobei die Breite der Räume zwischen nebeneinanderliegenden Windungen an dem distalen Ende der Hülse größer ist als diejenige an dem proximalen, neben dem Adapter (30) liegenden Ende.

9. Einführhülse nach einem der vorhergehenden Ansprüche, wobei der flache Drahtstreifen abgeschrägte oder abgerundete Kanten hat.

10. Einführhülse nach Anspruch 9, wobei die abgeschrägten oder abgerundeten Kanten des Drahtstreifens in jeder Windung der Spule im wesentlichen jenen der angrenzenden Windungen benachbart sind.

11. Einführhülse nach einem der Ansprüche 1 bis 10, wobei die Metallspule (12) mit einem dichten, strahlungsundurchlässigen Metall plattiert ist.

12. Einführhülse nach einem der Ansprüche 1 bis 11, wobei die innere Oberfläche der Metallspule mit einer Schmierumhüllung versehen ist.

13. Einführhülse nach einem der vorhergehenden Ansprüche, wobei die äußere Umhüllung (20) der Hülse eine vorgeformte Kunststoffmuffe aufweist, die auf der Metallspule (12) wärmegeschrumpft ist.

14. Einführhülse nach einem der vorhergehenden Ansprüche, wobei die Dicke der Kunststoffumhüllung in dem Bereich von 0,0254 bis 0,203 mm (0,001 und 0,008 Inch) liegt.

15. Einführhülse nach einem der vorhergehenden Ansprüche, wobei die Kunststoffumhüllung auf ihrer äußeren Oberfläche eine glatte Bearbeitung hat und die Bearbeitung durch spitzenloses Schleifen der äußeren Oberfläche geschaffen ist.

16. Einführhülse nach einem der vorhergehenden Ansprüche, wobei die Kunststoffumhüllung eine äußere Schmieroberfläche hat.

17. Einführhülse nach einem der vorhergehenden Ansprüche, wobei die Hülse an dem Ende eine Metallspitze (60) hat.

18. Einführhülse nach Anspruch 17, wobei die Metallspitze (60) aus einem strahlungsundurchlässigen Metall hoher Dichte besteht oder dieses aufweist.

19. Einführhülse nach Anspruch 11 oder 18, wobei das strahlungsundurchlässige Metall Tantal oder Gold ist.

## Revendications

1. Gaine d'introduction (10) destinée à être introduite par voie percutanée dans un vaisseau d'un corps humain ou animal et, lorsqu'elle est ainsi introduite, servant de guide pour l'introduction ultérieure d'un cathéter dans ledit vaisseau, la gaine (10) comprenant un adaptateur (30) placé à une extrémité de la gaine pour permettre l'introduction de fils métalliques de guidage et/ou de cathéters par ladite gaine et dans ledit vaisseau, ainsi qu'un manchon d'introduction (12, 20) comprenant un enroulement de fil métallique (12) constitué d'une bande de fil plat qui forme au moins une partie de la paroi intérieure du manchon, la bande étant revêtue ou recouverte d'une enveloppe de matière plastique (20) qui est placée sur et qui est en contact avec la surface extérieure dudit enroulement métallique, caractérisée en ce que :
ledit adaptateur (30) comprend une valve d'hémostase ; et
ledit enroulement de fil métallique (12) est réalisé de manière à comprendre des espaces entre ses spires voisines et ladite enveloppe de matière plastique (20) comprend des parties (25, 26, 27, 28) qui pénètrent dans lesdits espaces sur une distance suffisant à lui permettre de résister à un déplacement longitudinal relatif desdites spires voisines lorsque ladite gaine est courbée, mais sans recouvrir la surface intérieure de l'enroulement métallique.

2. Gaine d'introduction selon la revendication 1 ou 2, dans laquelle ledit enroulement métallique (12) est réalisé en acier inoxydable.

3. Gaine d'introduction selon la revendication 1 ou la revendication 2, dans laquelle le fil plat de l'enroulement métallique a une épaisseur de l'ordre de 0,0635 à 0,0889 mm (2,5 à 3,5 millièmes de pouce) et un rapport de la largeur à l'épaisseur de l'ordre de 3:1 à 20:1 ou une épaisseur de l'ordre de 0,0381 à 0,0632 mm (1,50 à 2,49 millièmes de pouce) et un rapport de la largeur à l'épaisseur de l'ordre de 5:1 à 50:1 ou une épaisseur de l'ordre de 0,019 à 0,0378 mm (0,75 à 1,49 millième de pouce) et un rapport de la largeur à l'épaisseur de l'ordre de 12:1 à 80:1.

4. Gaine d'introduction selon l'une quelconque des revendications 1 à 3, dans laquelle chacun des espaces séparant les spires voisines de l'enroulement est plus petit que la largeur d'une unique spire de l'enroulement.

5. Gaine d'introduction selon l'une quelconque des revendications 1 à 3, dans laquelle les espaces séparant les spires voisines de l'enroulement sont plus grands que la largeur d'une spire unique de l'enroulement.

6. Gaine d'introduction selon la revendication 4 ou 5, dans laquelle les espaces séparant les spires voisines sont tous de la même largeur.

7. Gaine d'introduction selon la revendication 4 ou 5, dans laquelle la largeur des espaces séparant les spires voisines varie sur la longueur de l'enroulement.

8. Gaine d'introduction selon la revendication 7, dans laquelle la largeur des espaces séparant les spires voisines à l'extrémité distale de la gaine est plus grande que celle les séparant à l'extrémité proximale voisine dudit adaptateur (30).

9. Gaine d'introduction selon l'une quelconque des revendications précédentes, dans laquelle la bande plate de fil comporte des bords chanfreinés ou arrondis.

10. Gaine d'introduction selon la revendication 9, dans laquelle les bords chanfreinés ou arrondis de la bande de fil sont sensiblement contigus, dans chaque spire de l'enroulement, de ceux des spires voisines.

11. Gaine d'introduction selon l'une quelconque des revendications 1 à 10, dans laquelle l'enroulement métallique (12) comporte un placage de métal dense radio-opaque.

12. Gaine d'introduction selon l'une quelconque des revendications 1 à 11, dans laquelle la surface intérieure de l'enroulement métallique est munie d'un revêtement lubrifiant.

13. Gaine d'introduction selon l'une quelconque des revendications précédentes, dans laquelle l'enveloppe extérieure (20) de la gaine consiste en un manchon préformé de matière plastique qui a subi un rétrécissement à la chaleur sur l'enroulement métallique (12).

14. Gaine d'introduction selon l'une quelconque des revendications précédentes, dans laquelle l'épaisseur de l'enveloppe de matière plastique est de l'ordre de 0,0254 mm à 0,203 mm (0,001 à 0,008 pouce).

15. Gaine d'introduction selon l'une quelconque des revendications précédentes, dans laquelle l'enveloppe de matière plastique a un fini lisse sur sa surface extérieure, le fini étant produit par polissage au tonneau de la surface extérieure.

16. Gaine d'introduction selon l'une quelconque des revendications précédentes, dans laquelle l'enveloppe de matière plastique a une surface extérieure lubrifiée.

17. Gaine d'introduction selon l'une quelconque des revendications précédentes, dans laquelle la gaine comporte un bout métallique (60) à l'extrémité.

18. Gaine d'introduction selon la revendication 17, dans laquelle le bout métallique (60) consiste en ou comprend un métal radio-opaque de densité élevée.

19. Gaine d'introduction selon la revendication 11 ou la revendication 18, dans laquelle le métal radio-opaque est du tantale ou de l'or.
